# EUROPEAN PATENT SPECIFICATION

(11) **EP 1 751 292 B1**
(45) Date of publication and mention of the grant of the patent: **07.07.2010**
(21) Application number: 04751134.0
(22) Date of filing: 03.05.2004
(51) Int. Cl.: C12P 7/64

(54) **A METHOD TO PRODUCE SUCCINIC ACID FROM RAW HYDROLYSATES**
VERFAHREN ZUR HERSTELLUNG VON BERNSTEINSÄURE AUS ROHHYDROLYSATEN
PROCÉDÉ DE FABRICATION D'ACIDE SUCCINIQUE À PARTIR D'HYDROLYSATS BRUTS

(43) Date of publication of application: 14.02.2007
(73) Proprietor: UT-Battelle, LLC, Oak Ridge, TN 37831-6498 (US); UChicago Argonne, LLC, Chicago, Illinois 60637 (US)
(72) Inventor: NGHIEM, Nhuan, Phu, Knoxville, TN 37923 (US); DONNELLY, Mark, Warranville, IL 60555 (US); SANVILLE-MILLARD, Cynthia, Y., Plainfield, IL 60544 (US)
(74) Representative: Ferreccio, Rinaldo
(86) International application number: PCT/US2004/013605
(87) International publication number: WO 2005/116227

(56) References cited:
- WO-A-2004/043881
- US-A1- 2003 017 559
- NGHIEM ET AL: "Production of succinic acid from lignocellulosic materials" SLIDES / ORAL PRESENTATION AT THE 221ST MEETING OF THE ACS, 2001, pages 1-24, XP002490031
- VEMURI ET AL: "Succinate production in dual-phase Escherichia coli fermentations depends on the time of transition from aerobic to anaerobic conditions" JOURNAL OF INDUSTRIAL MICROBIOLOGY, vol. 28, 2002, pages 325-332, XP003009348
- ANONYMOUS: KGB004 BIOCHEMICAL AND CHEMICAL PLANT DESIGN - FALL 2004, LULEÂ UNIVERSITY OF TECHNOLOGY, SWEDEN, [Online] 2004, pages 1-16, XP002490032 Retrieved from the Internet: URL:www.km.luth.se/ugst/chemit/kgb010/KGB0 04LECT1.pdf> [retrieved on 2008-07-29]
- ANONYMOUS: INTERNET ARTICLE, [Online] 2001, pages 1-4, XP002490033 Retrieved from the Internet: URL:www.km.luth.se/ugst/chemit/kgb010/KGB0 04%20BACKGROUND%20ON%20SUCCINIC%20ACID%20P ROCESS.pdf> [retrieved on 2008-07-29]
- VEMURI ET AL: 'Effects of Growth Mode and Pyruvate Carboxylase on Succinic Acid Production by Metabolically Engineered Strains of Escherichia coli' APPL ENVIRON MICROBIOL vol. 68, no. 4, April 2002, pages 1715 - 1727, XP002987293
- CHATTERJEE ET AL: 'MUTATION OF THE PTSG GENE RESULTS IN INCREASED PRODUCTION OF SUCCINATE IN FERMENTATION OF GLUCOSE BY ESCHERICHIA COLI' APPL ENVIRON MICROBIOL vol. 67, no. 1, January 2001, pages 148 - 154, XP000996310
- LEE ET AL: 'Biological conversion of wood hydrolysate to succinic acid by Anaerobiospirillum succiniciproducens' BIOTECHNOL LETT. vol. 25, no. 2, January 2003, pages 111 - 114, XP002987294

## Description

### BACKGROUND OF THE INVENTION

### 1. Field of the Invention

This invention relates to a fermentation method to produce succinic acid, and to certain bacterial mutants capable of utilizing a myriad of sugars to produce succinic acid as a major fermentation product.

### 2. Background of the Invention

Carboxylic acids hold promise as potential precursors for numerous chemicals. For example, succinic acid can serve as a feedstock for such plastic precursors as 1,4 butanediol (BDO) tetrahydrofuran, and gamma-butyroactone. New products derived from succinic acid are under development, with the most notable of these being polyester which is made by linking succinic acid and BDO. Generally, esters of succinic acid have the potential of being new, "green" solvents that can supplant more harmful solvents. In total, succinic acid could serve as a precursor for millions of pounds of chemicals annually at a total market value of over $1 billion. Along with succinic acid, other 4-caton dicarboxylic acids, such as malic acid, and fumario acid also have feedstock potential.

The production of these carboxylic acids from renewable feedstocks (in this case through fermentation processes) is an avenue to supplant the more energy intensive methods of deriving such acids from nonrenewable sources. Succinate is an intermediate for anaerobic fermentations by propionate-producing bacteria but those processes result in low yields and concentrations.

Anaerobic rumen bacteria, such as *Bacteroides ruminicola* and *Bacteroides amylophilus* also produce succinate. However, rumen organisms are characteristically unstable in fermentation processes.

It has been long been known that a mixture of acids are produced from *E*. *coli* fermentation, as elaborated in Stokes, J.L. 1949 "Fermentation of glucose by suspensions of Escherichia coli" J. Bacteriol. 57:147-158. However, for each mole of glucose fermented, only 1.2 moles of formic acid, 0.1-02 moles of lactic acid, and 0.3-0.4 moles of succinic acid are produced. As such, efforts to produce carboxylic acids fermentatively have resulted in relatively large amounts of growth substrates, such as glucose, not being converted to the desired product.

Some bacteria, such as *A*. *succiniciproducens*, utilized in fermentation processes as outlined in U.S. Patent No. 5,143,834 to Glassner et al., naturally produce succinic acid in moderate liters up to only about 35-40 grams per liter (g/L). The *A. succiniciproducens* host strain has been shown to be not highly osmotolerant in that it does not tolerate high concentrations of salts and is further inhibited by moderate concentrations of product. Lastly, *A. succiniciproducens* presents handling in that as an obligate anaerobe, procedures using the organism must be done in the absence of oxygen. Also, medium preparation for the inoculum, requires the addition of tryptophan.

Previous efforts by the inventors to produce succinic acid has resulted in the isolation and utilization of a mutant bacterium. The mutant, available as ATCC accession number 202021, is the subject of U.S , Patent Reissue Application No. 09/429,693 (US RE37,3936; Sep 25, 2001). Reissue Application No. 09/429,693, teaches a succinic acid-producing bacterial stain (AFP 111) which spontaneously mutates from its precursor. The mutant is able to grow fermentatively on glucose to produce succinic acid in high yields, while its precursors are unable to do so. However, an obvious drawback to utilizing this method of succinic acid production is its limitation to a single mutant.

Other efforts (U.S. Patent No. 6,159,738) by the inventors have resulted in a method for constructing bacterial strains having increased succinic acid production. The method teaches that alteration of the phosphotransferase gene of E. coli causes the bacteria to produce more succinic acid. A drawback to this method is its limitation to a single alteration.

A need exists in the art for a method for producing succinic acid fermentatively, whereby the method is not relegated to a single mutant or gene. The method should be enabled by any organism having a particular, and easily determined, genotype. The method should be able to be performed in relatively inert conditions using robust organisms (i.e., those having high feed back inhibition thresholds), and also so as to obviate the need for sophisticated environmental control measures. The method should produce superior results utilizing mixtures of sugars derived from hydrolysis of lignocellulosic materials, inasmuch as these substrates offer a cheaper source of sugars, and as such, their use could reduce production costs for succinic acid.

US 2003/017559 discloses a method for producing succinic acid from industrial-grade hydrolysates, comprising supplying an organism that contains mutations for the genes ptsG, pflB, and ldhA, allowing said organism to accumulate biomass, and allowing said organism to metabolize the hydrolysate. While certain specific E. coli mutants (AFP 184 and AFP 400) are mentioned, none of them were accessible to the public at the time of publication of US 2003/017559.

The above method according to US 2003/017559, in which the E. coli mutant AFP 184 is used, is also known from the oral presentation of Nghiem et al. "Production of succinic acid from lignocellulosic materials" at the 221st Meeting of the ACS, 2001, pages 1-24.

Vemuri et al., "Succinate production in dual-phase Escherichia coli fermentations depends on the time of transition from aerobic to anaerobic conditions", Journal of Microbiology, Vol. 28, 2002, pages 325-332, disclose a method for producing succinic acid from media based on glucose, using the Escherichia coli mutant AFP 111. A similar method is also disclosed in Vemuri et al., Appl. Environ. Microbiol. 2002 Apr, 68(4): 1715-27.

### SUMMARY OF THE INVENTION

It is an object of the present invention to provide a method of producing succinio acid that overcomes many of the disadvantages of the prior art.

It is another object of the present invention to provide a fermentation process that produces high yields of succinic acid.

Yet another object of the present invention is to produce succinic acid fermentatively.

Briefly a method of producing succinic acid from industrial-grade hydrolysates according to claim 1 is provided.

Also provided is a bacteria mutant according to claim 9.

### BRIEF DESCRIPTION OF THE DRAWING

The present invention together with the above and other objects and advantages may best be understood from the following detailed description of the embodiment of the invention illustrated in the drawings, wherein:
FIG. 1 is a graph depicting an enhanced production of succinic acid after transformation of a bacteria with a mutant gene, in accordance with features of the present invention;
FIG. 2 is a graph depicting fermentation of industrial hydrolysate via a triple mutant organism, in accordance with features of the present invention; and
FIG. 3 is a graph depicting fermentation of synthetic sugar via a triple mutant organism, in accordance with features of the present invention.

### DETAILED DESCRIPTION OF THE INVENTION

The inventors have developed a method for fermentatively producing high yields of succinic acid. The method exploits altered catabolite repression mechanisms of selected organisms so as to allow the organisms to produce succinic acid using mixtures of glucose and non-glucose feedstocks.

Prior to setting forth the invention in detail, the following definitions if appearing herein, are provided:

A "vector" is a replicon, such as plasmid, phage or cosmid, to which another DNA segment may be attached so as to bring about the replication of the attached segment.

A "replicon" is any genetic element (e.g., plasmid, chromosome, virus) that functions as an autonomous unit of DNA replication *in vivo*, i.e., capable of replication under its own control.

A "cassette" refers to a segment of DNA that can be inserted into a vector at specific restriction sites. The segment of DNA encodes a polypeptide of interest, and the cassette and restriction sites are designed to ensure insertion of the cassette in the proper reading frame for transcription and translation.

A cell has been "transfected" by exogenous or heterologous DNA when such DNA has been introduced inside the cell. A cell has been "transformed" by exogenous or heterologous DNA when the transfected DNA effects a phenotypic change.

"Heterologous" DNA refers to DNA not naturally located in the cell, or in a chromosomal site of the cell. Preferably, the heterologous DNA includes a gene foreign to the cell.

A "nucleic acid molecule" refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms. Thus, this term includes double-stranded DNA found, *inter alia*, in linear or circular DNA molecules (e.g., restriction fragments), plasmids, and chromosomes. In discussing the structure of particular double-stranded DNA molecules, sequences may be described herein according to the normal convention of giving only the sequence in the 5' to 3' direction along the non-transcribed strand of DNA (i.e., the strand having a sequence homologous to the mRNA). A "recombinant DNA molecule" is a DNA molecule that has undergone a molecular biological manipulation.

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength (see Sambrook *et al., supra*). The conditions of temperature and ionic strength determine the "stringency" of the hybridization. For preliminary screening for homologous nucleic acids, low stringency hybridization conditions, corresponding to a Tₘ of 55°C, can be used, e.g., 5X SSC, 0.1% SDS, 0.25% milk, and no formamide; or 30% formamide, 5X SSC, 0.5% SDS). Moderate stringency hybridization conditions correspond to a higher Tₘ, e.g., 40% formamide, with 5X or 6X SCC. High stringency hybridization conditions correspond to the highest Tₘ, e.g., 50% formamide, 5X or 6X SCC. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tₘ for hybrids of nucleic acids having those sequences. The relative stability (corresponding to higher Tₘ) of nucleic acid hybridizations decreases in the following order: RNA:RNA, DNA:RNA, DNA:DNA. For hybrids of greater than 100 nucleotides in length, equations for calculating Tₘ have been derived (see Sambrook *et al., supra*, 9.50-0.51). For hybridization with shorter nucleic acids, i.e., oligonucleotides, the position of mismatches becomes more important, and the length of the oligonucleotide determines its specificity (see Sambrook *et al., supra*, 1.1.7-11.8). Preferably a minimum length for a hybridizable nucleic acid is at least about 12 nucleotides; preferably at least about 18 nucleotides: and more preferably the length is at least about 27 nucleotides; and most preferably about 36 nucleotides.

"Homologous recombination" refers to the insertion of a foreign DNA sequence of a vector in a chromosome. Preferably, the vector targets a specific chromosomal site for homologous recombination. For specific homologous recombination, the vector will contain sufficiently long regions of homology to sequences of the chromosome to allow complementary binding and incorporation of the vector into the chromosome. Longer regions of homology, and greater degrees of sequence similarity, may increase the efficiency of homologous recombination.

A DNA "coding sequence" is a double-stranded DNA sequence which is transcribed and translated into a polypeptide in a cell *in vitro* or *in vivo* when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic MRNA, genomic DNA sequences from eukaryotic (e.g., mammalian) DNA, and even synthetic DNA sequences. If the coding sequence is intended for expression in a eukaryotic cell, a polyadenylation signal and transcription termination sequence will usually be located 3' to the coding sequence.

Transcriptional and translational control sequences are DNA regulatory sequences, such as promoters, enhancers, terminators, and the like, that provide for the expression of a coding sequence in a host cell. In eukaryotic cells, polyadenylation signals are control sequences.

A "promoter sequence" is a DNA regulatory region capable of binding RNA polymerase in a cell and initiating transcription of a downstream (3' direction) coding sequence. For example, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined for example, by mapping with nuclease S1), as well as protein binding domains (consensus sequences) responsible for the binding of RNA polymerase.

A coding sequence is "under the control" of transcriptional and translational control sequences in a cell when RNA polymerase transcribes the coding sequence into mRNA, which is then trans-RNA spliced and translated into the protein encoded by the coding sequence.

As used herein, the term "sequence homology" in all its grammatical forms refers to the relationship between proteins that possess a "common evolutionary origin," including proteins from superfamilies (e.g., the immunoglobulin superfamily) and homologous proteins from different species (e.g., myosin light chain, etc.) [Reeck et al., Cell, 50:667 (1987)].

Accordingly, the term "sequence similarity" in all its grammatical forms refers to the degree of identity or correspondence between nucleic acid or amino acid sequences of proteins that do not share a common evolutionary origin [see Reeck et al., 1987, supra]. However, in common usage and in the instant application, the term "homologous," when modified with an adverb such as "highly," may refer to sequence similarity and not a common evolutionary origin.

Two DNA sequences are "substantially homologous" or "substantially similar" when at least about 50% (preferably at least about 75%, and most preferably at least about 90%, 95% or 99.9%) of the nucleotides match over the defined length of the DNA sequences. Sequences that are substantially homologous can be identified by comparing the sequences using standard software available in sequence data banks, or in a Southern hybridization experiment under, for example, stringent conditions as defined for that particular system. Defining appropriate hybridization conditions is within the skill of the art. See, e.g., Maniatis *et al., supra*; DNA Cloning, Vols. I & II, supra; Nucleic Acid Hybridization, *supra*.

Similarly, two amino acid sequences are "substantially homologous" or "substantially similar" when greater than 30% of the amino acids are identical, or greater than about 60% are similar (functionally identical). Preferably, the similar or homologous sequences are identified by alignment using, for example, the GCG (Genetics Computer Group, Program Manual for the GCG Package, Version 7, Madison, Wis.) pileup program.

The term "corresponding to" is used herein to refer similar or homologous sequences, whether the exact position is identical or different from the molecule to which the similarity or homology is measured. Thus, the term "corresponding to" refers to the sequence similarity, and not the numbering of the amino acid residues or nucleotide bases.

The resulting mutants and protocols result in a succinate to feedstock ratio of up to 1.3:1, and typically 0.9:1. Succinate accumulations of between 60 g/L and 75 g/L are achieved. Typical protocol durations are more than 70 hours, and usually between 120 and 170 hours. For example yields of 70 g/L are obtained after 160 hours. The process is viable at from between about 25 °C and 45 °C, with a preferable range of about 30 to 39 °C. A pH of between about 5 and 9 is suitable, with a more preferable range of about 6.1 and 7.2.

The invented mutants are especially viable components of the fermentative protocol inasmuch as they have increased tolerance to fermentative products. For example, concentrations of 72 g/L for succinate, 22 g/L for acetate, 14 g/L for ethanol, and 8 g/L for lactate are achievable without inducing feedback inhibition.

### Feedstock Detail

A salient feature of the invented method and mutant is the direct utilization of industrial feedstocks. A myriad of feedstocks can be utilized, including, but not limited to light steep water, lignocellulosic hydrolysate produced by various methods of hydrolysis, corn-derived sugar solutions (such as corn steep liquor), lactose from whey, and other industrial-grade sugars. For example, lignocellulosic hydrolysate produced by concentrated acid hydrolysis, or dilute acid hydrolysis, enzyme hydrolysis or hydrolysates produced by a combination of these processes are all suitable. Corn-derived sugar solutions are also suitable.

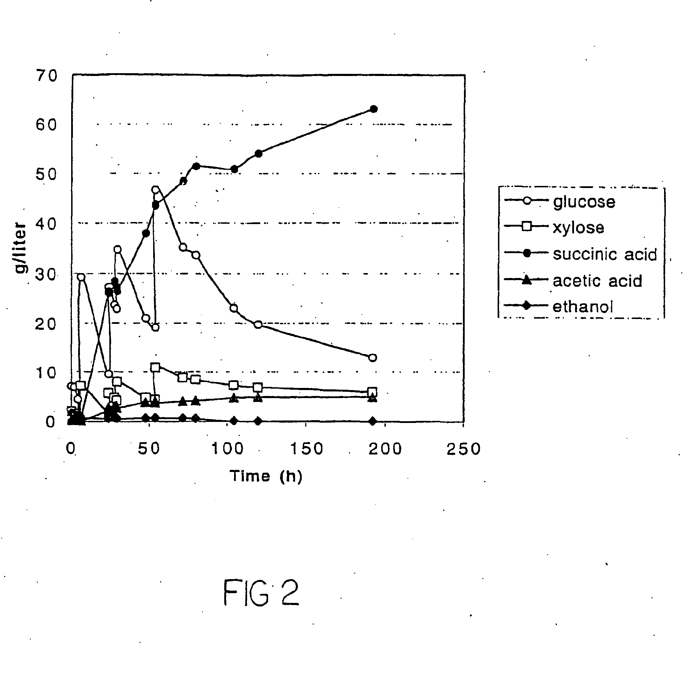

Industrial feedstocks generally are mixtures of glucose and other sugars, the most common non-glucose sugar being xylose. FIG. 2 depicts the utilization of glucose and xylose by one of the invented mutants.

In light of the foregoing, any feedstocks containing glucose and/or non-glucose sugars are suitable. As such, feedstocks containing glucose, sorbitol, xylose, arabi-nose, mannose, lactose, glucuronic acid, galactose, fructose, and combinations thereof are appropriate.

### Organism Detail

The invented method utilizes organisms according to claim 9 containing alterations in the catabolite repression system of the organisms. Specifically, the inventors have found that, as alterations exist to the phosphotransferase (pts) system, pyruvate formate lyase (pfl) system, and lactate dehydrogenase (ldh) system of bacteria, these bacteria are suitable for use in the invented succinic acid producing process. *pfLAB* and *ldhA*, are the genes encoding pyruvate:formate lyase and the fermentative lactate dehydrogenase, respectively.

Organisms to be altered to include the three knockouts are modified by serial transduction using bacteriophage P1. Standard P1 transduction protocols were utilized, an exemplary protocol disclosed in J. H. Miller, ed. Experiments in Molecular Genetics 1972 (Cold Spring Harbor Laboratory, Cold Spring Harbor. N.Y.). Using this method, wild-type or near wild-type strains of bacteria (e.g., the C600 strain of *E. coli*; ATTC accession no. 23724) can be used to create mutant substrains that lack one, two, or three functional genes selected from *pfl, ldh, ptsG*.

"Gene knockout" refers to a process of silencing the expression of a particular gene in a cell. The silencing process may include, for example, gene targeting or antisense blocking. Gene targeting refers to a process of introducing a nucleic acid construct into a cell to specifically recombine with a target gene. The nucleic acid construct inactivates the targeted gene. Inactivation may be by introduction of termination codons into a coding region or introduction of a repression site into a regulatory sequence. Antisense blocking refers to the incorporation into a cell of expression sequences which directs the synthesis of antisense RNA to block expression of a target gene. Antisense RNA hybridizes to the mRNA of the target gene to inhibit expression.

One example of an *E. coli* strain comprising three mutations that could be used in the invention was named AFP 184 (ATCC accession number PTA 5132, deposited April 9, 2003) (AFP=Alternative Feedstock Program). AFP 184 has the pfl deletion, 1dh knockout, and the different mutant form of ptsG deliberately inserted into a near wild-type strain of *E. coli.* Another strain called AFP 415 can also be used. AFP 415 differs from AFP 184 only in having the knockout of ptsG. It performs similarly to AFP 184.

Surprisingly and unexpectedly, the inventors found that the metabolism rate and titer for AFP 184 and AFP 415 were superior to the W1485 derivatives disclosed in U.S. Patent Nos. 5,770,435 (now Reissue Application No. 09/429,693) and 6,159,738.

Table 1 provides a comparison of succinic acid production by AFP 184 and a W1485 derivative (AFP 111). It is noteworthy that while the W1485 derivative utilized fairly refined feedstocks, AFP 184 still provided higher values with industrial grade hydrolysates.

A mutation containing all three knockouts also can be generated using a bacterium already containing one or two of the genetic anomalies, and then inducing the remainder knockout(s). In this instance, a viable starting organism is W1485, ATCC Accession Number 12435. AFP 400 (ATCC accession number PTA 5583, deposited October 10, 2003), is a deliberately-made triple knockout. It contains the pfl deletion by August Bock, and inserted into W1485 by David Clark of the University of Illinois to produce FMJ123. FMJ123 is produced pursuant to the protocol found in P.K. Bunch et al. (1997) Microbiology 143, 187-195. AFT 400 also contains the 1dhA knockout, and inserted into FMJ123 to produce DC1327. DC1327 is produced pursuant to the protocol found in Chatterjee et al, Appl. Environ. Microbiol. 67, pp148-154, and incorporated herein by reference. AFP 400 contains the *ptsG* knockout, as described in the Chatterjee reference.

**Table 1: Comparison of Succinic Acid Production by Different Lineages Of E.Coli**

| Strain | Max Concentration | Max Productivity | Yield (g/g glucose) |
|---|---|---|---|
| AFP111 | 51 g/L | 0.87 g/Lh | 0.70 |
| AFP 184 | 72 g/L | 1.00 g/Lh | 1.00 |

A triple knockout AFP404 (ATCC accession number PTA 5133, deposited April 9, 2003) was also constructed by introduction of three knockouts into strain C600. AFP404 is similar to AFP 184 but has a knockout of *ptsG* rather than a point mutation of the gene. It also produces succinic acid in a yield of approximately 1 mol/mol glucose.

A protocol for development of the triple mutation from the wild strain also is found in R. Chatterjee et al. Typical antibiotic markers indicating presence of each of the knockouts include, but are not limited to, Cam, Tet, and Kan. New *E. coli* strains, AFP 400 and AFP 404 containing the knockouts and the antibiotic markers were thus generated. That protocol follows:

### Construction and introduction of an insertionally inactivated ptsG gene

The native *ptsG* gene of *E. coli* was cloned by PCR from genomic DNA prepared from W1485 using primers targeting the N- and C- termini of the protein with no additional genomic sequences amplified. The gene was cloned in the vector pFJ118EH to give pJFptsG. The gene was disrupted by insertion of the kanamycin resistance cassette of pUC-4K (Pharmacia), excised with EcoRI, into the MfeI site of the ptsG gene in pJFptsG to give the plasmid pTSGK. Because NZN 111 already includes a kanamycin resistance marker, an equivalent stain was constructed by transducing Tn10-inactivated 1dhA gene from stain SE1752 into FMJ123. The resulting strain, DC1327, was indistinguishable in its physiology from NZN 111. The disrupted ptsG gene was transferred in DC1327 by transforming the cells with pTSGK, growing the cells for approximately 30 generations in the presence of kanamycin and absence of ampicillin, then plating the culture on LB plates containing glucose and incubating anaerobically. Colonies that were able to grow fermentatively were purified and screened for their sensitivity to the two antibiotics, as described in detail in the Examples which follow.

Strain AFP400 was isolated as a stable kanamycin resistant, ampicillin sensitive strain that fermented glucose to succinate, acetate, and ethanol. Proper integration of the disrupted *ptsG* gene was confirmed by PCR. The disrupted gene was amplified from AFP400 DNA using primers that matched flanking sequences approximately 110 base pairs outside the coding region of the gene. These sequences were not present in the integration vector. The resulting product was 3.0 kb in size, as predicted from the known sequence *ptsG*, its flanking regions, and the Kanamycin insert. The product was digested with ClaI (site in the kanamycin cassette) and AgeI (site in ptsG), and generated the fragments expected for insertion of the cassette into the MfeI site of ptsG (1.95 and 1.05 kb for ClaI, and 2.3 and 0.7 kb for Agel).

Yet another strain comprising the three knock outs, AFP 404, is also derived from C600, a near wild-type E. coli K12 strain, using the same protocol above.

Location of the knockouts are already known from the inventor's previous research (U.S. Patent No. 6,159,738, and Chatterjee et al.) discussed *supra*. The knockouts are introduced using a copy of the knock-out gene, having a resistance marker, for transforming cells. Homologous recombination is allowed to occur, as facilitated by host enzymes. The chromosome containing the marker is then selected. The ptsG knockout was introduced this way. Proof of its insertion, via PCR, is detailed in Chatterjee, et al.,.

### Growth Detail

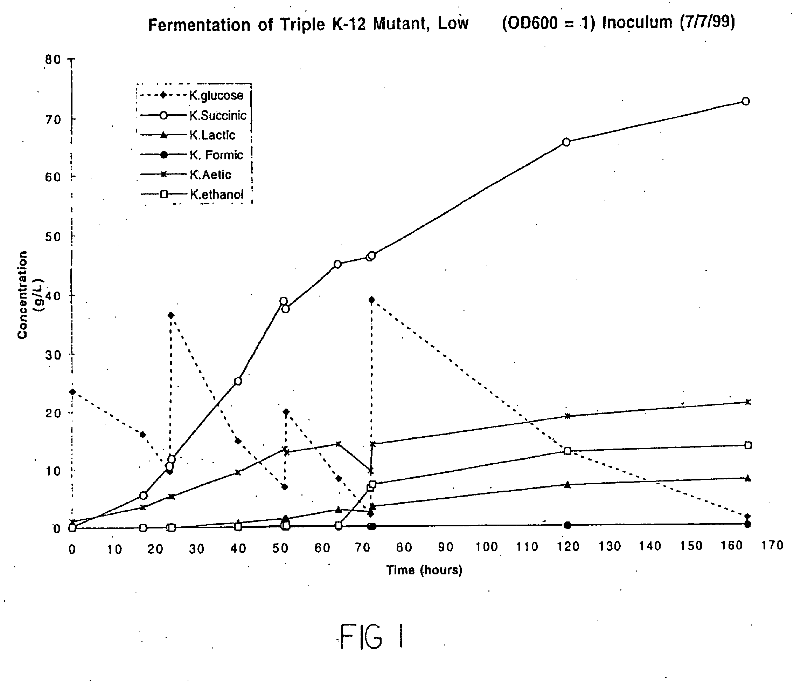

The triple mutant organisms produced by the inventors are not obligate anaerobes. As such, initial accumulation of biomass can occur aerobically, after which fermentative conditions are established. The advantages of this two-stage process (i.e., aerobic-then anaerobic) protocol are illustrated in FIG. 2 wherein the rate of production of succinic acid is much larger compared to the single-stage anaerobic protocol growth curve of FIG. 1.

Generally, when the biomass reaches a point of the equivalent of approximately 10⁸ to 10¹¹ cells per milliliter (or approximately 2 to 5 gram dry cell weight per liter), the fermenter is made anaerobic. In the laboratory, this concentration point was reached after approximately six hours.

In industrial protocols, a fermenter is charged with light steep water plus lignocellulosic hydrolysate. Antibiotics were included as necessary at the following concentrations: 100 µg of carbenicillin per ml, 30 µg of kanamycin per ml, 10 µg of tetracycline per ml, and 30 µg of chloramphenicol per ml. Rich broth contained (per liter), 10 g of tryptone, 5 g of NaCl, and 1 g of yeast extract. Solid media for plates contained 1.5 percent (wt/vol) Difco Bacto-Agar. Minimal medium E was prepared as described in Vogel, H.J. 1956 Acetylornithinase in E. coli, . Biol. Chem. 218:97-103.

### Laboratory conditions for the fermentation were as follows:

Fermentative growth was performed in sealed serum tubes containing 10 ml of LB medium, supplemented with 0.5 g of MgCO₃ (added in order to maintain the pH of the medium during fermentation), antibiotics, and approximately 10 g/L of glucose. A myriad of growth substrates can be utilized, including but not limited to sugars, sugar alcohols, sugar acids and combinations thereof. The following sugars were tested in place of glucose at a concentration of 5 g/L in anaerobic growth: trehalose, mannose, fructose, sorbitol, and glucuronic acid.

Innocula for the anaerobic liquid cultures were prepared by growing the strains aerobically overnight in LB medium supplemented with antibiotic. A sample of the overnight culture was diluted 100-fold in fresh media and allowed to grow aerobically to an A600 of approximately 1; the anaerobic growth media was inoculated with 1 ml of the innocula.

Samples were removed anoxically from the sealed tubes at appropriate times for analysis of levels of glucose (or alternate sugar substrates) remaining and fermentation products formed. For anaerobic growth on solid media, agar plates were incubated at 37°C in an anaerobic jar under an H₂-CO₂ atmosphere generated by use of a Gas-Pak.

A plate assay for β-galactosidase activity was used to test for the presence of normal catabolite repression in strains. LB or Medium E-agar are two of several mediums which can be utilized. Medium E-agar is a minimum-nutrient medium commonly used, and discussed in Vogel, H.J., 1956 Acetylornithase in E. coli, J. Biol. Chem 218:97-103. In exemplary protocols, LB or Medium E-agar is supplemented with 4 g/L of glucose, 4 g/L of lactose, 3 mg/L of 5-bromo-4-chloro-3-indolyl- β-D-galactoside (X-gal), and antibiotics. These media are hereinafter referred to as X-gal/glucose agar. The formation of blue colonies indicated expression of β-galactosidase in the presence of glucose due to the absence of normal catabolite repression. Conversely, the formation of white colonies indicated that normal catabolite repression existed, and therefore no enzyme was present to cleave the disaccharide lactose.

The inventors also have devised a method for utilizing the mutant in a continuous process. In this continuous process, repetitive experiments were conducted in which after the culture had produced approximately 50 g/L succinic acid, one milliliter of the mixture was added to a fresh enclosure containing LB media, glucose and MgC03. This new innoculum continued to produce succinic acid effectively. This process was repeated 3-4 times, in each case resulting in efficient production of succinic acid.

### Example 1-Succinic Acid Production Utilizing Industrial Hydrolysate

AFP 184 was placed in a fermenter with true hydrolysate, from rice straw. An exemplary hydrolysate is that commercially prepared and made available from Arkenol Inc., of Mission Viejo, CA, via its concentrated acid hydrolysis process. The rice straw medium contains approximately 600 g/L glucose and 169 g/L xylose as the two main sugar components, plus minor quantities of other sugars. The experimental data are found in Table 2 and in FIG. 2.

The following is a protocol of the AFP 184-based fermentation process: The fermentation medium contained the following components: Difco yeast extract 5 g/L, tryptone 10 g/L, (NH₄)2SO₄ 2 g/L, MgSO₄-7H₂O 0.2 g/L, NaCl 10 g/L, K₂HPO₄ 7 g/L, KH₂PO₄ 3 g/L, Arkenol's hydrolysate 16.5 mg/L, and kanamycin 30 mg/L. The industrial hydrolysate contained 607 g/L glucose and 169 g/L xylose as the two main sugar components plus minor quantities of other sugars. The medium with all of the components except the antibiotic was autoclaved at 121°C for 20 minutes. Kanamycin then was added upon cooling. This fermentation medium was used for both the inoculum flasks and the one-liter fermenter. For the inoculum, 50 mg medium was placed in a 250-mg flask and inoculated with 0.2 mg of the AFP184 stock culture which was maintained in 30% glycerol and at -70°C. The flask was incubated in a incubator shaker at 37°C and 250 rpm overnight (about 16 hours). The entire flask contents then were used to inoculate the fermenter which was maintained at 37°C. The medium in the fermenter was aerated to allow fast growth of the organism. After six hours when the required cell mass was achieved, the following actions were taken: 1. Air was turned off to exert anaerobic conditions, which would initiate production of succinic acid; 2. Carbon dioxide gas was sparged into the medium at a rate of 0.03 mg per minute; and 3. A feed solution which contained the Arkenol's hydrolysate diluted with deionized water to a concentration of 500 g/L of total glucose plus xylose was added to the fermenter to achieve a total sugar concentration of 50 g/L in the fermentation medium. During the course of the experiment, when the sugar concentration in the fermenter was low, more feed was added to provide sufficient substrates for succinic acid production. As the cells produced succinic acid the pH dropped. It was maintained at pH 6.5 by addition of a 1.5 M Na₂CO₃ solution through the action of an automatic pH controller. Samples were taken at intervals and analyzed for optical density, glucose, xylose, succinic acid, acetic acid, lactic acid, and ethanol.

**Table 2: Production of Succinic Acid, Acetic Acid and Ethanol from Arkenol With a Mutant Containing ptsG, ldh, and pfl Anomalies**

| Time | glucose | xylose | succinic acid | acetic acid | ethanol |
|---|---|---|---|---|---|
| 0 | 7.04 | 1.94 | 0 | 0 | 1.60 |
| 2 | 6.85 | 1.53 | 0 | 0.41 | 1.35 |
| 4.2 | 4.41 | 0 | 0 | 0.85 | 1.13 |
| 6 | 0 | 0 | 0 | 0.55 | 1.04 |
| 6.05 | 29.27 | 7.17 | 0 | 0 | 0.68 |
| 24 | 9.56 | 1.69 | 26.12 | 2.24 | 0.49 |
| 24.05 | 27.25 | 5.60 | 26.39 | 2.82 | 0.72 |
| 28.1 | 23.7 | 14.69 | 28.42 | 2.98 | 0.71 |
| 29.5 | 22.8 | 14.17 | 27.20 | 3.04 | 0.67 |
| 29.55 | 34.77 | 7.95 | 26.41 | 2.63 | 0.52 |
| 48 | 20.98 | 4.72 | 37.98 | 3.71 | 0.62 |
| 54 | 19.13 | 4.30 | 43.82 | 4.10 | 0.71 |
| 54.05 | 46.73 | 10.85 | 43.51 | 3.69 | 0.59 |
| 72 | 35.14 | 8.85 | 48.52 | 4.01 | 0.63 |
| 80 | 33.60 | 8.45 | 51.44 | 4.10 | 0.50 |
| 104.25 | 23.02 | 7.20 | 50.99 | 4.64 | 0 |
| 120 | 19.73 | 6.77 | 54.12 | 4.83 | 0 |
| 192 | 13.04 | 5.87 | 63.21 | 4.88 | 0 |

### Example 2-Succinic Acid Production From Synthetic Sugar Mixture

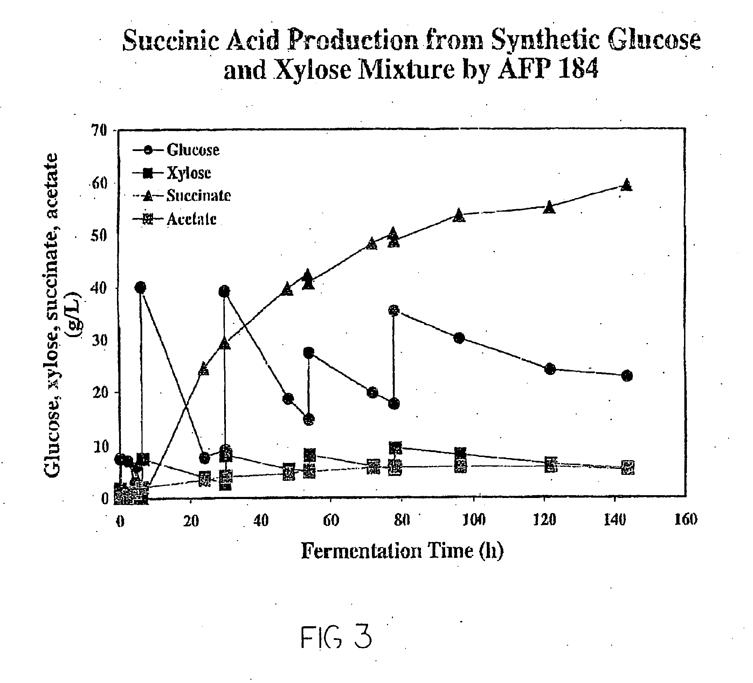

A fermentation protocol was developed utilizing AFP 184 in combination with a synthetic sugar feedstock. As can be noted on FIG. 3, succinate production was rapid up to 80 hours, and plateaued somewhat before reaching a final high of 60 g/L after approximately 140 hours.

The fermentation medium contained the following components: Difco yeast extract 5 g/L, tryptone 10 g/L, (NH₄)2SO₄ 2 g/L, MgSO₄-7H₂O.0.2 g/L, NaCl 10 g/L, K₂HPO₄ 7 g/L, KH₂PO₄ 3 g/L, glucose 7.6 g/L, xylose 1.85 g/L, and kanamycin 30 mg/L. The medium with all of the components except the antibiotic was autoclaved at 121°C for 20 minutes. Kanamycin then was added upon cooling. This fermentation medium was used for both the inoculum flasks and the one-liter fermenter. For the inoculum, 50 mg medium was placed in a 250-mg flask and inoculated with 0.2 mg of the AFP184 stock culture which was maintained in 30% glycerol and at -70°C. The flask was incubated in a incubator shaker at 37°C and 250 rpm overnight (about 16 hours). The entire flask contents then were used to inoculate the fermenter which was maintained at 37°C.

The medium in the fermenter was aerated to allow fast growth of the organism. After six hours when the required cell mass was achieved, the following actions were taken:
1. Air was turned off to exert anaerobic conditions, which would initiate production of succinic acid;
2. Carbon dioxide gas was sparged into the medium at a rate of 0.03 mg per minute; and
3. A feed solution which contained 400 g/L glucose and 84 g/L xylose was added to the fermenter to achieve a total sugar concentration of 50 g/L in the fermentation medium.

During the course of the experiment, when the sugar concentration in the fermenter was low, more feed was added to provide sufficient substrates for succinic acid production. As the cells produced succinic acid, the pH dropped. It was maintained at pH 6.5 by addition of a 1.5 M Na₂CO₃ solution through the action of an automatic pH controller. Samples were taken at intervals and analyzed for optical density, glucose, xylose, succinic acid, acetic acid, lactic acid, and ethanol.

Table 3, infra, and FIG. 3 illustrate the succinic acid production resulting from the utilization of the synthetic sugar mixture.

As can be noted in a comparison between Example 1 and Example 2, succinate production of the mutant was equivalent (see time points 120 and 122 of Table 2 and 3, respectively) when industrial hydrolysate was used versus when the synthetic feedstock was used. This result illustrates the robust character of the invented protocol in that any toxic materials inherent with industrial grade hydrolysates did not degrade the yield.

**Table 3: Succinic Acid Production in a fermentation protocol utilizing Synthetic Sugar**

| Time | Glucose | Xylose | Succinate | Acetate |
|---|---|---|---|---|
| 0 | 7.65 | 1.85 | 0 | 0 |
| 2 | 7.19 | 1.03 | 0 | 0.32 |
| 4.2 | 3.15 | 0 | 0 | 1.1 |
| 4.45 | 6.03 | 0.84 | 0 | 1.1 |
| 6 | 1.04 | 0 | 0 | 2.02 |
| 6.25 | 40.2 | 7.57 | 0 | 2.02 |
| 24 | 7.76 | 3.92 | 24.55 | 3.43 |
| 30 | 9.18 | 2.63 | 29.34 | 4.11 |
| 30.25 | 39.3 | 8.2 | 29.34 | 4.11 |

| Time | Glucose | Xylose | Succinate | Acetate |
|---|---|---|---|---|
| 48 | 18.6 | 5.5 | 39.8 | 4.6 |
| 54 | 14.8 | 4.95 | 42.33 | 5.26 |
| 54.25 | 27.4 | 8.1 | 40.77 | 4.9 |
| 72 | 19.7 | 6.04 | 48.33 | 5.76 |
| 78 | 17.6 | 5.42 | 50.27 | 6 |
| 78.25 | 35.5 | 9.49 | 48.87 | 5.75 |
| 96.5 | 30.2 | 8.25 | 53.62 | 5.87 |
| 122 | 24.1 | 6.48 | 55.1 | 5.87 |
| 144 | 22.8 | 5.67 | 59.35 | 5.43 |

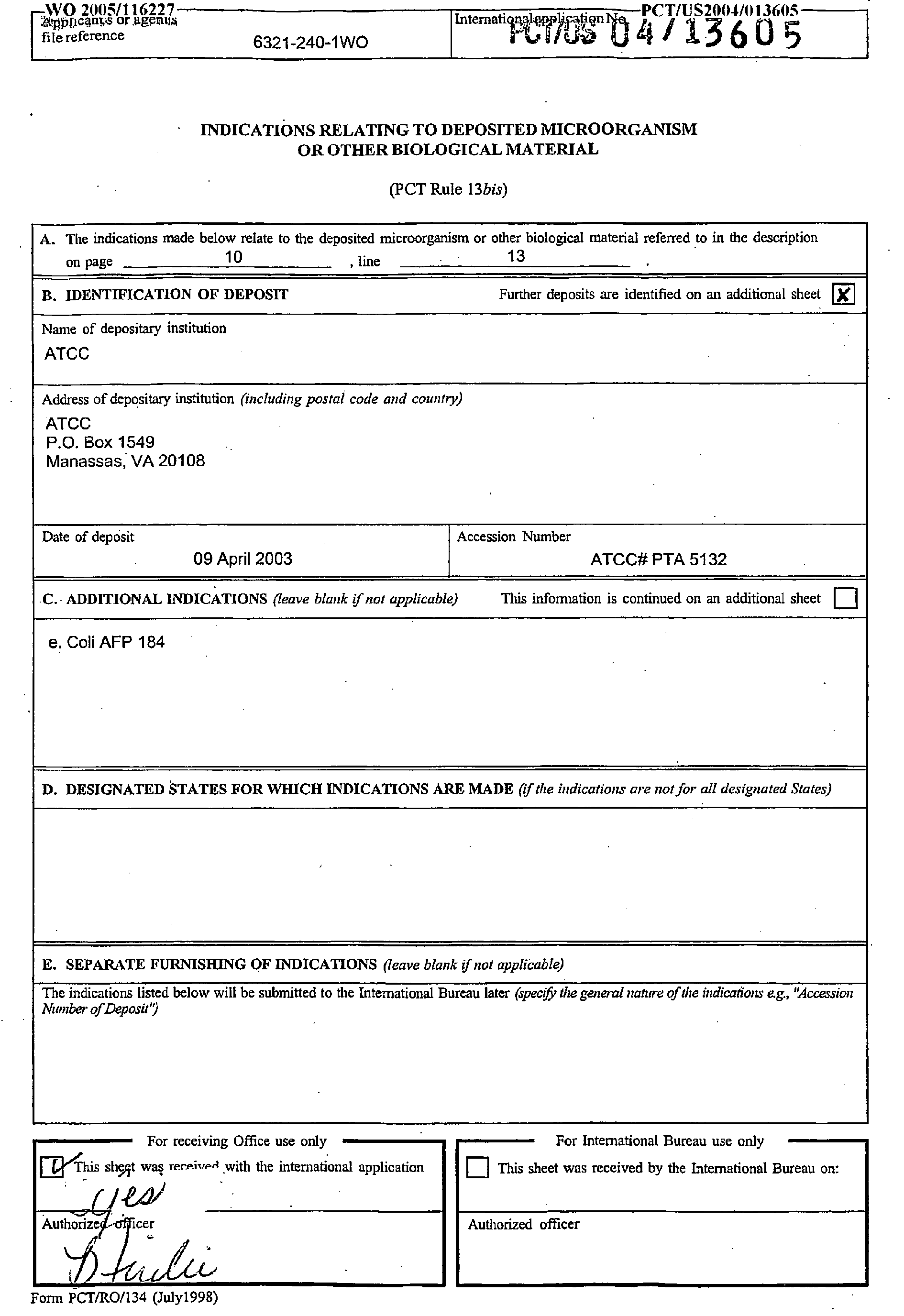

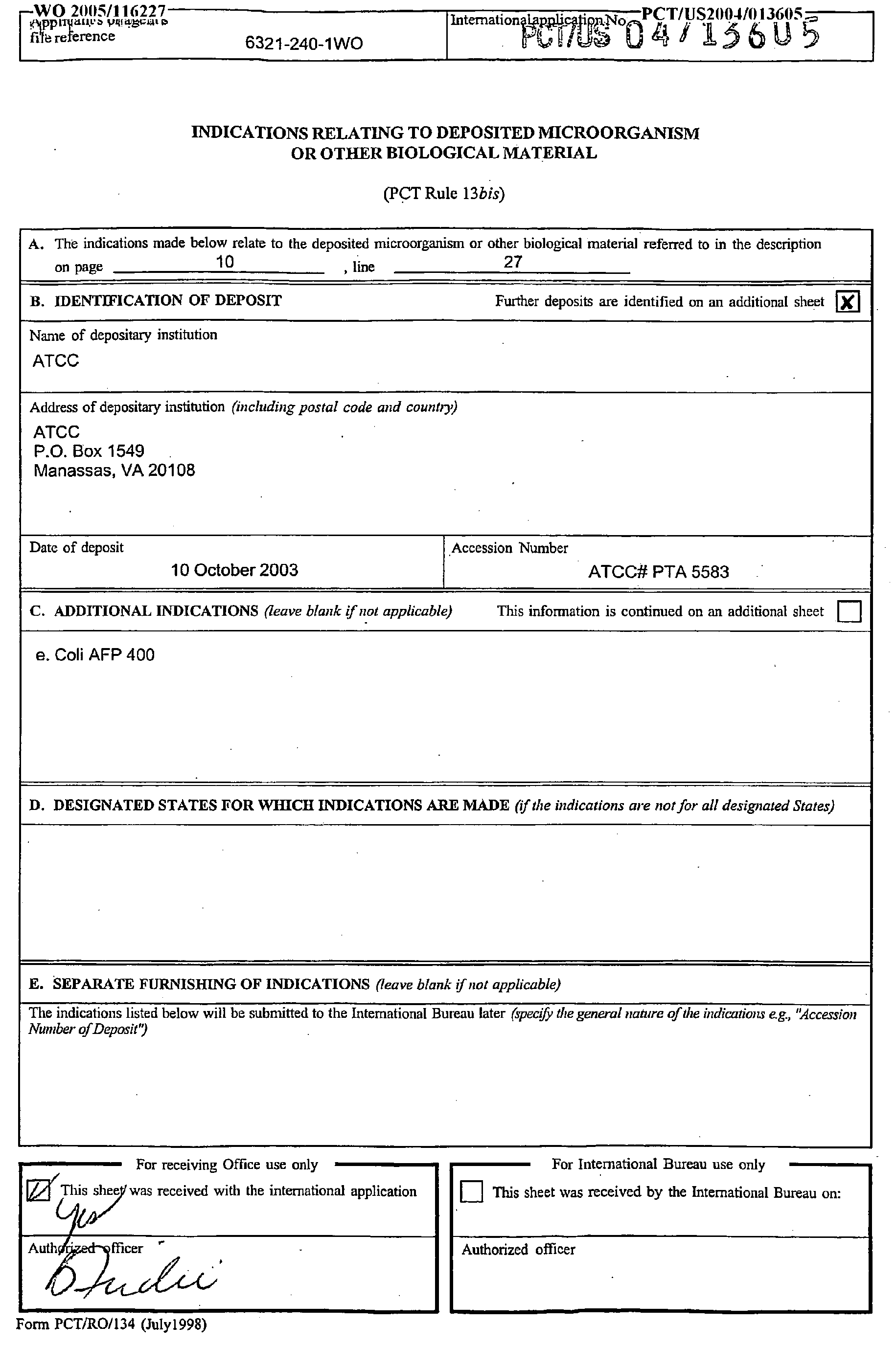

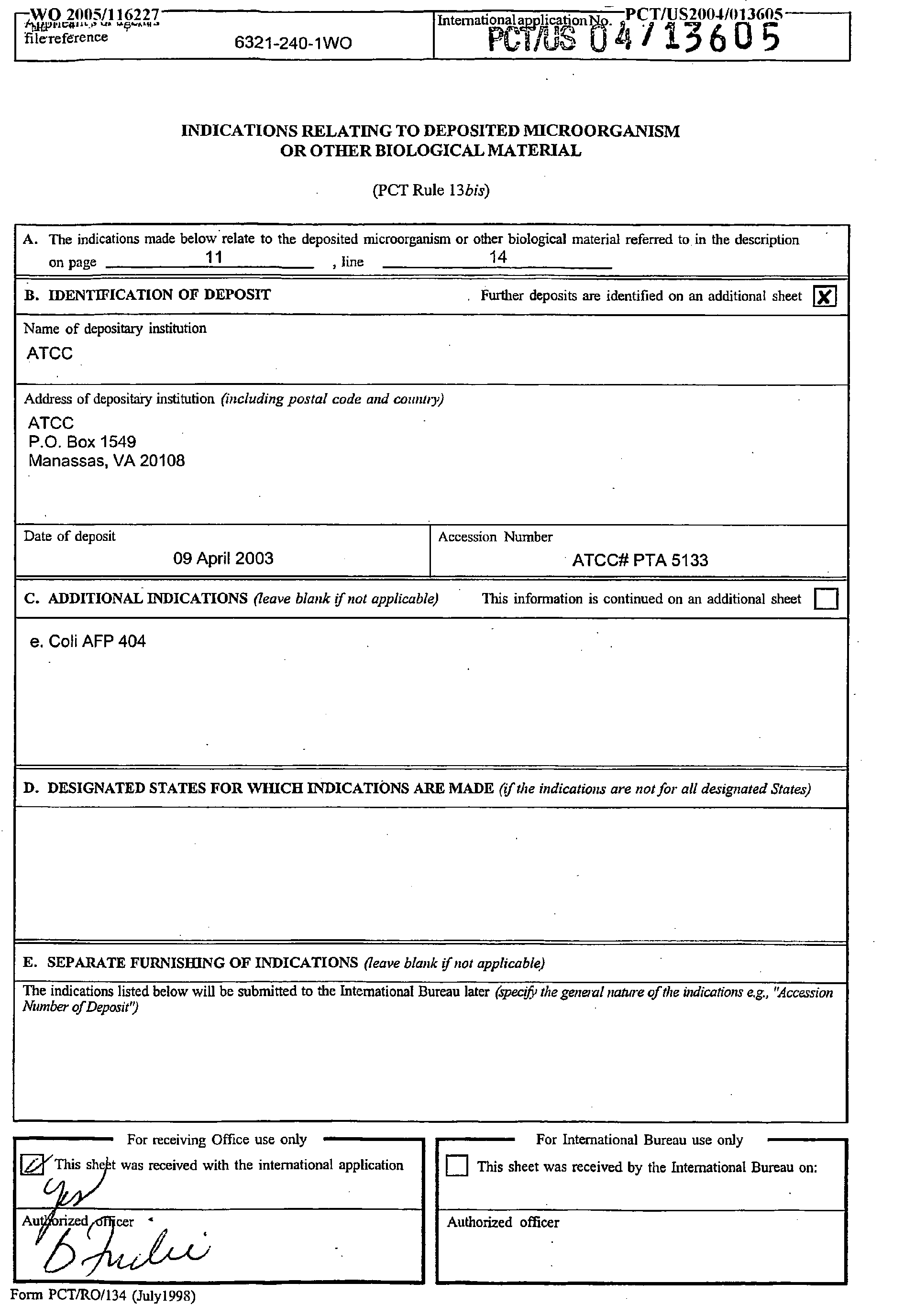

## Claims

1. A. method of producing succinic acid from industrial-grade hydrolysates comprising :
a) supplying an organism from an Escherichia coli strain selected from AFP 184 (ATCC PTA 5132), AFP 400 (ATCC PTA 5583) and AFP 404 (ATCC PTA 5133);
b) allowing said organism to accumulate biomass; and
c) allowing said organism to metabolize the hydrolysate.

2. The method as recited in claim 1 wherein the biomass accumulates to between approximately 10⁸ to10¹¹ cells per milliliter.

3. The method as recited in claim 1 wherein the industrial-grade hydrolysate is lignocellulosic hydrolysate, or corn-derived sugar solutions.

4. The method as recited in claim 1 wherein the temperature is selected from between approximately 25°C and 45°C.

5. The method as recited in claim 1 wherein the biomass accumulates in an aerobic atmosphere.

6. The method as recited in claim wherein the pH is selected from between approximately 5 and 9.

7. The method as recited in claim 1 wherein the hydrolysate is contained in a first feedstock amount and wherein the method is made continuous with the addition of a second feedstock amount.

8. The method as recited in claim 7 wherein the second feedstock amount is added when succinic acid concentration is approximately 50 g/ L.

9. An Escherichia coli strain mutant selected from AFP 184 (ATCC PTA 5132), AFP 400 (ATCC PTA 5583) and AFP 404 (ATCC PTA 5133), **characterized in that** it produces succinic acid from substrate contained in industrial-grade hydrolysate in a ratio of between 0.6:1 and 1.3:1 succinic acid to substrate.

10. The mutant as recited in claim 9 wherein the substrate is a sugar selected from the group consisting of glucose, lactose, sorbitol, xylose, arabinose, mannose, glucuronic acid, galactose, fructose, or combinations thereof.

11. The mutant as recited in claim 9 wherein the mutant is capable of utilizing more than one substrate simultaneously to produce succinic acid simultaneously.

## Patentansprüche

1. Verfahren zur Herstellung von Bernsteinsäure aus Hydrolysaten industrieller Qualität, umfassend:
a) Bereitstellen eines Organismus aus einem *Escherichia coli*-Stamm ausgewählt aus AFP 184 (ATCC PTA 5132), AFP 400 (ATCC PTA 5583) und AFP 404 (ATCC PTA 5133);
b) Anhäufung von Biomasse durch den Organismus; und
c) Metabolisierung der Hydrolysate durch den Organismus.

2. Verfahren gemäß Anspruch 1, wobei sich die Biomasse auf ungefähr 10⁸ bis 10¹¹ Zellen pro Milliliter anhäuft.

3. Verfahren gemäß Anspruch 1, wobei die Hydrolysate industrieller Qualität entweder lignozellulosehaltige Hydrolysate oder Zuckerlösungen, die aus Getreide erhalten wurden, sind.

4. Verfahren gemäß Anspruch 1, wobei die Temperatur aus ungefähr 25°C bis 45°C ausgewählt wird.

5. Verfahren gemäß Anspruch 1, wobei sich die Biomasse in einer aeroben Atmosphäre anhäuft.

6. Verfahren gemäß Anspruch 1, wobei der pH aus ungefähr 5 bis 9 ausgewählt wird.

7. Verfahren gemäß Anspruch 1, wobei das Hydrolysat in einer ersten Rohstoffmenge enthalten ist und, wobei das Verfahren kontinuierlich mit der Zugabe einer zweiten Rohstoffmenge durchgeführt wird.

8. Verfahren gemäß Anspruch 7, wobei die zweite Rohstoffmenge dann zugegeben wird, wenn die Bernsteinsäurekonzentration bei ungefähr 50 g/L liegt.

9. *Escherichia coli*-Mutante ausgewählt aus AFP 184 (ATCC PTA 5132), AFP 400 (ATCC PTA 5583) und AFP 404 (ATCC PTA 5133), **dadurch gekennzeichnet, dass** sie Bernsteinsäure aus einem Substrat herstellt, das in einem Hydrolysat industrieller Qualität in einem Verhältnis von 0.6:1 bis 1.3:1 Bernsteinsäure zu Substrat enthalten ist.

10. Mutante gemäß Anspruch 9, wobei das Substrat ein Zucker ausgewählt aus der Gruppe bestehend aus Glucose, Lactose, Sorbitol, Xylose, Arabinose, Mannose, Glucuronsäure, Galactose, Fructose oder Kombinationen davon.

11. Mutante gemäß Anspruch 9, wobei die Mutante dazu fähig ist, mehr als ein Substrat gleichzeitig zu verwenden, um gleichzeitig Bernsteinsäure herzustellen.

## Revendications

1. Une méthode de production d'acide succinique à partir d'hydrolysats de qualité industrielle, la méthode comprenant les étapes de :
a) fournir un organisme dérivé d'une souche Escherichia coli choisi dans le groupe constitué par les souches AFP 184 (ATCC PTA 5132), AFP 400 (ATCC PTA 5583) et AFP 404 (ATCC PTA 5133);
b) permettre audit organisme d'augmenter sa biomasse; et
c) permettre audit organisme de métaboliser l'hydrolysat.

2. La méthode selon la revendication 1 **caractérisée en ce que** la biomasse atteint une concentration variant entre approximativement 10⁸ à 10¹¹ cellules par millilitre.

3. La méthode selon la revendication 1 **caractérisée en ce que** l'hydrolysat de qualité industrielle est un hydrolysat lignocellulosique ou une solution de sucre dérivée de maïs.

4. La méthode selon la revendication 1 **caractérisée en ce que** la température varie entre approximativement 25° et 45° C.

5. La méthode selon la revendication 1 **caractérisée en ce que** la biomasse augmente en milieu aérobie.

6. La méthode selon la revendication 1 **caractérisée en ce que** le pH varie entre approximativement 5 et 9.

7. La méthode selon la revendication 1 **caractérisée en ce que** l'hydrolysat est contenu dans une première matière première et où la méthode est en mode continu avec l'addition d'une deuxième matière première.

8. La méthode selon la revendication 7 **caractérisée en ce que** la deuxième matière première est ajoutée quand la concentration d'acide succinique est approximativement de 50 g/L.

9. Une souche de Escherichia coli mutante choisie dans le groupe constitué par AFP 184 (ATCC PTA 5132), AFP 400 (ATCC PTA 5583) et AFP 404 (ATCC PTA 5133), **caractérisée en ce qu'**elle produit de l'acide succinique à partir d'un substrat contenu dans un hydrolysat de niveau industriel dans un ratio entre 0.6:1 et 1.3 :1 d'acide succinique et de substrat.

10. La souche mutante selon la revendication 9 **caractérisée en ce que** le substrat est un sucre sélectionné à partir du groupe constitué par le glucose, le lactose, le sorbitol, le xylose, l'arabinose, le mannose, l'acide glucuronique, le galactose, le fructose ou une combinaison de ceux-ci.

11. La souche mutante selon la revendication 9 **caractérisée en ce que** la souche mutante est capable d'utiliser plus qu'un substrat simultanément pour produire l'acide succinique.
